# EUROPÄISCHE PATENTSCHRIFT

(11) **EP 0 309 602 B1**
(45) Veröffentlichungstag und Bekanntmachung des Hinweises auf die Patenterteilung: **24.03.1993**
(21) Anmeldenummer: 87114289.9
(22) Anmeldetag: 30.09.1987
(51) Int. Cl.: A61K 37/54

(54) **Verwendung katabolischer Enzyme zur Herstellung eines Medikaments zum Bekämpfen der erworbenen Immunschwäche (AIDS) und deren Vorstadien (LAS, ARC)**
Use of catabolic enzymes for the manufacture of a medicament in the treatment of AIDS and its early stages (LAS, ARC)
Utilisation d'enzymes catabolisantes pour la fabrication d'un médicament destiné au traitement du syndrome d'immunodéficience acquis (SIDA) et leurs stades préliminaires (LAS, ARC)

(43) Veröffentlichungstag der Anmeldung: 05.04.1989
(73) Patentinhaber: MUCOS EMULSIONSGESELLSCHAFT m.b.H., D-13509 Berlin (DE)
(72) Erfinder: Ransberger, Karl, D-8022 Grünwald (DE); Stauder, Gerhard, Dr., D-8190 Wolfratshausen (DE)
(74) Vertreter: Grünecker, Kinkeldey, Stockmair & Schwanhäusser Anwaltssozietät

(56) Entgegenhaltungen:
- ROTE LISTE, 1986, Nr. 85075; "Wobe-Mugos"
- ROTE LISTE, 1986, Nr. 85074; "Wobe-Mugos"
- ROTE LISTE, 1986, Nr. 41001; "Wobenzym"

## Beschreibung

Die Erfindung bezieht sich auf die Verwendung katabolischer Enzyme zur Herstellung eines Medikaments zum Bekämpfen der erworbenen Immunschwäche (AIDS) und deren Vorstadien (LAS,ARC).

Die genannten Krankheitsstadien umfassen die Stadien WR1 bis WR6 gemäß der Walter-Reed-Klassifikation. Die Abkürzungen LAS und ARC stehen dabei für "Aids-related-complex" beziehungsweise "Lymphadenopathiesyndrom".

Die erworbene Immunschwäche AIDS und deren Vorstadien (LAS, ARC) ist eine Viruserkrankung. Sie geht auf ein Virus zurück, das zur Gruppe der im Tierreich weit verbreiteten Retroviren gehört und Humane-Immundeficience-Virus (HIV) genannt wird. Aus der HIV-Infektion resultieren Störungen der gesamten Immunabwehrmechanismen, da vor allem die Helferzellen des T-Systems der spezifischen Immunabwehr und damit Schlüsselpositionen der Immunabwehr betroffen sind. Die durch die HIV-Infektion betroffenen T4- oder Helferzellen werden daran gehindert, ihre zentrale Rolle bei der Regulation der Immunantwort zu erfüllen. Der Störeffekt durch die HIV-Virusinfektion kann offensichtlich in einer direkten oder indirekten Reduktion der genannten Zellen bestehen.

Nach HIV-Infektionen werden Störungen der Balance zwischen den verschiedenen T-Zellpopulationen beobachtet, wobei insbesondere das Gleichgewicht zwischen T4- und T8-, zwischen Helfer- und Suppressorzellen, gestört zu sein scheint. Das T4/T8-Verhältnis, das normalerweise 1,4 bis 2,0 beträgt, ist zugunsten von T8-Zellen verschoben. Es kehrt sich um, so daß ein T4/T8-Verhältnis von 1:2 oder noch geringerer Werte auftritt. Die HIV-induzierte, persistierende T4-Zellen-Verminderung hat zur Folge, daß bei Patienten, bei denen das geschilderte Mißverhältnis zwischen T4- und T8-Zellen vorliegt, Erreger, die bei Vorliegen einer normalen Balance zwischen den verschiedenen T-Zellpopulationen harmlos sind, günstige Milieubedingungen antreffen. Die Erreger können Viren, Bakterien, Protozoen oder Pilze sein. Diese sogenannten opportunistischen Infektionen bewirken bei HIV-Patienten sehr unterschiedliche Krankheitsbilder. Das AIDS-Vollbild schließt neben der HIV-Infektion opportunistische, persistierende oder rezidivierende Krankheiten ein, die auf Defekte des T-Immunsystems hinweisen. Häufig werden verschiedene, von unterschiedlichen Erregern ausgelöste Entzündungserkrankungen gleichzeitig bei AIDS-Erkrankten angetroffen.

Weiterhin werden krebsartige Gewebsentartungen beobachtet, die ansonsten sehr selten auftreten, beispielsweise das sogenannte Kaposi-Sarkom, sowie andere maligne Erkrankungen, wie beispielsweise Lymphome des Gehirns, Non-Hodgkin- und Burkitt-Lymphome.

Die bei AIDS-Vollbild-Patienten nachweisbare Lymphopenie (<1000/mm³), der T-Helferzellabbau (<400/mm³), die Anämie, Leuko- und Thrombozytopenie, die fehlende In-vitro-Stimulierbarkeit der Lymphozyten sowie die fehlende Reaktivität gegenüber "Recall"-Antigenen sind objektive Kriterien für die Störung des Retikuloendothelial-Systems (RES).

Bei AIDS-Vollbild-Patienten findet man weiterhin gesteigerte Immunglobulin-Titer der IgA, IgG und IgM.

Es gibt weiterhin Hinweise darauf, daß der Immunkomplex (IK)-Spiegel bei AIDS-Erkrankten erhöht sein kann. Nach Lightfoote et al.(1985) sind für AIDS-Erkrankte insbesondere IgA-haltige Immunaggregate kennzeichnend.

Die Pathogenität der Immunkomplexe beruht auf deren Interaktion mit verschiedenen Komponenten der spezifischen und unspezifischen Immunabwehr. Zu dieser gehören neben der Inhibition der Makrophagen die Stimulation von T-Suppressor-Zellen, die thrombogenen und erythrozytenaggregierenden sowie insbesondere die komplementaktivierenden Eigenschaften, durch die es zu Entzündungserkrankungen und Gewebsläsionen kommt.

Ob es sich bei AIDS und deren Vorstadien (LAS, ARC) um Autoaggressions- oder Immunkomplex-Erkrankungen auf der Basis einer zytotoxischen Reaktion, nämlich einer allergischen Reaktion vom Typ II,handelt, ist derzeit nicht entschieden.

Die zahlreichen Publikationen über die ätiologischen Mechanismen bei AIDS und deren Vorstadien (LAS,ARC) sind nicht ohne Widersprüche. Wie aus der Darstellung von Rieber (1986) hervorgeht, hat das AIDS-Virus mehrere Möglichkeiten, in zentrale Funktionen des Immunsystems einzugreifen.

Die therapeutischen Bemühungen bei der Behandlung der Primärerkrankung, dem T4-Zellabbau, blieben bislang erfolglos. Eine kausale Behandlung der HIV-Infektion und der sich daraus ableitenden Immunschwäche ist im Augenblick klinisch nicht möglich. Die derzeit praktizierten Behandlungen konzentrieren sich einerseits auf die Hemmung der Virusvermehrung und andererseits auf die bei AIDS und deren Vorstadien (LAS,ARC) anzutreffenden Folgeerkrankungen, nämlich die opportunistischen Infektionen und die malignen Prozesse (Goebel et al., 1985).

Obwohl bereits mehr als 100 Substanzen (Koch, 1987) zur Bekämpfung der initialen Virusinfektion und der durch die damit verbundenen Folgeerkrankungen geradezu fieberhaft in aller Welt eingesetzt und getestet wurden, ist der bisher erzielte Erfolg entmutigend.

Der Erfindung lag die Aufgabe zugrunde, eine wirksame Bekämpfung der erworbenen Immunschwäche (AIDS) und deren Vorstadien (LAS, ARC) und den damit verbundenen Folgeerkrankungen zur Verfügung zu stellen.

Die Aufgabe wird dadurch gelöst, daß katabolische Enzyme zur Herstellung eines Medikaments zum Bekämpfen der erworbenen Immunschwäche (AIDS) und deren Vorstadien (LAS, ARC) und den damit verbundenen Folgeerkrankungen eingesetzt werden.

Immunkomplexe werden pathogen, wenn sie sich an Zellen binden. Die Immundeffizienz wird synergistisch intensiviert, wenn hierdurch die Aktivität von Makrophagen und natürlichen Killerzellen (NK) reduziert wird. Durch die somit steigende Konzentration von Immunkomplexen wird die Aktivität von Makrophagen weiter blockiert.

Die Verwendung katabolischer Enzyme, insbesondere hydrolytischer oder proteolytischer Enzyme, zur Therapie verschiedener Krankheiten ist seit den 50er Jahren bekannt, wobei der Einsatz der Enzyme bei der Behandlung von akuten und chronischen Entzündungen, bei Zirkulationsstörungen und in der Tumortherapie beschrieben wurde.

Wenngleich der In-vitro-Abbau von löslichen Immunkomplexen durch hydrolytische oder proteolytische Enzyme bekannt war, war es dennoch überraschend, daß durch die erfindungsgemäße Verwendung der bekannten katabolischen Enzyme die tatsächlich beobachtete, signifikante Verbesserung des Krankheitszustands bei AIDS-Patienten eintrat.

Durch die erfindungsgemäß verwendeten katabolischen Enzyme werden Immunkomplexe verdaut, indem der Fc-Arm der Antikörper geschnitten wird. Bei der noch ungeklärten Ätiologie der Immunschwächekrankheit war die daraus resultierende Verbesserung der gesamten Immunlage des Patienten dennoch nicht zu erwarten. Eine Erklärung für den beobachteten positiven Effekt mag darin liegen, daß durch die Eliminierung der Immunkomplexe durch die erfindungsgemäß verwendeten Enzyme eine Verbesserung des T4/T8-Verhältnisses erreicht wird, wodurch die körpereigene Abwehr die pathogenen Antigene (das HIV-Virus oder dessen Bestandteile) wirksamer bekämpfen kann. Die Viren enthaltenden Zellen können dann durch die natürlichen Killerzellen besser eliminiert werden.

Durch die verwendeten hydrolytischen oder proteolytischen Enzyme werden Makrophagen und natürliche Killerzellen direkt aktiviert, wodurch die Immunabwehr noch zusätzlich verstärkt wird.

Ein weiteres bekanntes Wirkprinzip der verwendeten Enzyme beruht auf der Spaltung von makromolekularen Stoffwechselprodukten und nekrotisch entstandenem Zelldetritus.

Die überraschend positive Wirkung der Verwendung der erfindungsgemäß verwendeten Enzyme mag darauf beruhen, daß die durch die Immunkomplexe bedingte Blockade der immunregulatorischen Mechanismen sowie die gestörte Phagozytose der Zellen des Retikuloendothelial-Systems (RES-Zellen) aufgehoben wird. Es kommt durch die Enzymtherapie somit zur Modulation und Stimulation der spezifischen und unspezifischen Immunabwehr.

Die verwendeten Enzyme haben weiterhin fibrinolytische Eigenschaften, durch die es zur Zerstörung der Fibrinummantelung kommt, mit deren Hilfe zirkulierende Immunkomplexe zum Sedimentieren gebracht werden und sich so besser als sekundär gewebefixierte Immunkomplexe anheften können. In diesem Vorgang liegt die eigentliche Pathogenität der Immunkomplexe, denn die angehefteten, gewebefixierten Immunkomplexe können die Komplementkaskade bis zur Endphase, dem zytolytischen Komplex, aktivieren.

Aufgrund dieser hydrolytischen Remobilisierungsphänomene, durch die auch primär gewebeständige Immunaggregate abgebaut werden, kommt es zu einem Rückgang des Entzündungsgeschehens. Bei dem Immundefektsyndrom AIDS liegt ein hochkomplexes Entzündungsgeschehen durch unterschiedlichste Immunaggregate vor. Wie Jäger et al. 1987 an Walter Reed (WR)-klassifizierten HIV-positiven Patienten zeigten, nimmt der mittlere Immunkomplex-Titer bei verschiedenen WR-Stadien signifikant zu. Es wird ein Maximum an Immunkomplextitern erreicht. Bei finalen AIDS-Patienten ist auch das humorale Immunsystem geschwächt, wodurch die Immunkomplextiter wieder abfallen.

Die geschilderten Beobachtungen sind jedoch nicht klar interpretierbar und zeigen eine sehr komplexe Interaktion von Immunaggregaten verschiedener Herkunft. Zum Teil enthalten die Immunkomplexe HIV oder Bestandteile von HIV, die auf den HIV-Befall zurückgehen. Auch durch die opportunistischen Infektionen und malignen Wachstumsprozesse können die Immunkomplexe entstanden sein.

Neben der bemerkenswerten therapeutischen und unerwarteten Wirkung der Verwendung der erfindungsgemäßen Enzyme hat diese Verwendung weiterhin den Vorteil, daß auch bei Langzeitanwendung keine schädigenden Nebenwirkungen auftreten, wie durch die Verwendung der genannten Enzyme in den bereits erprobten Bereichen bekannt ist.

Vorzugsweise kann es sich bei den erfindungsgemäß verwendeten Enzymen um Papain, Pankreatin, Bromelain, Lipase, Amylase, Irypsin, α-Chymotrypsin oder Serratia-Peptidase handeln.

Papain ist ein proteolytisches, bis zu den Aminosäuren abbauendes Enzym, das aus dem Milchsaft der unreifen, fleischigen Früchte des Melonenbaums Carica papaya gewonnen wird.

Pankreatin wird aus Schweine- oder Rinderpankreas gewonnen.

Bromelain ist ein proteolytisch wirksames Enzym aus dem Preßsaft von Ananas.

Lipasen gehören zur Untergruppe der Esterasen und werden aus Pankreas oder dem Pilz Rhizopus arrhizus gewonnen.

Amylasen sind Glykosid-spaltende Enzyme, die beispielsweise aus Pankreas oder speziellen Mikroorganismen isoliert werden.

Trypsin und α-Chymotrypsin sind proteolytische Enzyme, die ebenfalls im Pankreas gebildet werden und in Verbindung mit anderen Enzymen bereits therapeutisch eingesetzt wurden.

Serratia-Peptidase kann aus einem Mikroorganismus der Gattung Serratia gewonnen werden.

Rutin, das der Enzymmischung weiterhin zugesetzt werden kann, gehört zu den Flavonoidglykosiden.

Kalbsthymusfaktoren, die ebenfalls gegebenenfalls den verwendeten Enzymmischungen zugesetzt werden können, werden aus Thymusdrüsen isoliert.

Die besondere Wirksamkeit der geschilderten Substanzen zeigt sich, wenn zwei oder mehr der genannten Enzyme in Kombination verwendet werden.

Der endgültig zur Anwendung kommenden Konfektionierung können Hilfsstoffe, wie sie üblicherweise verwendet werden, vorzugsweise Mannitol, zugegeben werden.

Besondere Wirksamkeit hat eine Mischung gezeigt, die aus fraktionierten Hydrolysaten von Rinderpankreas, Kalbsthymus, Pisum sativum und Lens esculenta gewonnen werden, sowie Papain und Mannitol, und zwar in den jeweiligen Mengen von 1-10, vorzugsweise 8 mg, 1-10, vorzugsweise 4 mg, 1-10, vorzugsweise 4 mg, 1-10, vorzugsweise 4 mg, 1-10, vorzugsweise 2 mg und 10-100, vorzugsweise 78 mg.

Eine weitere Ausführungsform der erfindungsgemäßen Enzymmischung enthält ein Gemisch von 10-30, vorzugsweise 24 mg Trypsin, 1-10, vorzugsweise 1 mg, α-Chymotrypsin, 40-100, vorzugsweise 60 mg Papain, 50-200, vorzugsweise 100 mg Pankreatin, 20-100, vorzugsweise 45 mg Bromelain, 5-50, vorzugsweise 10 mg Lipase, 5-50, vorzugsweise 10 mg Amylase sowie 10-100, vorzugsweise 50 mg Rutin.

Eine weitere Ausführungsform besteht aus einer Mischung von Trypsin, α-Chymotrypsin, Papain und Kalbsthymus in den jeweiligen Mengen von 1-10, vorzugsweise 4 mg, 1-10, vorzugsweise 4 mg, 5-50, vorzugsweise 10 mg und 1-10, vorzugsweise 3 mg in Ampullen oder jeweils 10-100, vorzugsweise 40 mg, 10-100, vorzugsweise 40 mg, 50-500, vorzugsweise 100 mg und 10-100, vorzugsweise 40 mg in Klistiertabletten oder oral einzunehmenden Tabletten.

Mit den geschilderten Enzymmischungen wird ein Therapieweg für die Behandlung der Immunschwäche AIDS und deren Vorstadien (LAS, ARC) aufgezeigt, der völlig neu ist und unerwartet positive Ergebnisse gezeigt hat.

Dies wird im folgenden anhand von klinischen Versuchen näher erläutert.

### Durchführung klinischer Versuche

| WOBE-MUGOS^{R} Enzymmischung I | Ampullen | Klistier-Tabl. | oral-Tabl. | WOBENZYM^{R} Enzymmischung II |
|---|---|---|---|---|
| enthaltene Enzyme | 1 Ampulle | 1 Tabl. | 1 Tabl. | 1 Dragee |
| Trypsin | 4 mg | 40 mg | 40 mg | 24 mg |
| α-Chymotryp- | 4 mg | 40 mg | 40 mg | 1 mg |
| sin | | | | |
| Papain | 10 mg | 100 mg | 100 mg | 60 mg |
| Kalbsthymus | 3 mg | 40 mg | 40 mg | ------ |
| Pankreatin | ----- | ------ | ------ | 100 mg |
| Bromelain | ----- | ------ | ------ | 45 mg |
| Lipase | ----- | ------ | ------ | 10 mg |
| Amylase | ----- | ------ | ------ | 10 mg |
| Rutin | ----- | ------ | ------ | 50 mg |

Es wurden Patienten behandelt, die einen positiven Test für HIV zeigen und klinische Symptome und/oder Anzeichen von AIDS, ARC (Aids-related-complex) oder LAS (Lymphadenopathiesyndrom) zeigen.

Die Tests für HIV wurden entsprechend den internationalen Standards durchgeführt, und die Patienten wurden ausgewählt, wenn mindestens zwei verschiedene Tests einen positiven Nachweis für die HIV-Infektion zeigten.

Dosierung und Applikation:
- 1.-4. Woche:: - bis zu 3 x wöchentlich 1 Ampulle WOBE-MUGOS^{R} pro Injektion
- an Tagen ohne Injektion bis zu 15 Tabletten WOBE-MUGOS^{R} pro Tag
- alternativ bis zu 15 WOBE-MUGOS^{R} Klistier-Tabletten rektal;
- begleitend bis zu 30 Dragees WOBENZYM^{R} pro Tag, (d.h. bis zu 10 Dragees 3 x täglich oder 6 Dragees 5 x täglich, usw.).
von der
- 5. Woche an:: - täglich bis zu 15 Tabletten WOBE-MUGOS^{R} pro Tag;
- alternativ bis zu 5 WOBE-MUGOS^{R} Klistier-Tabletten rektal;
- begleitend bis zu 30 Dragees WOBENZYM^{R} pro Tag (z.B. bis zu 10 Dragees 3 x täglich oder 6 Dragees 5 x täglich, usw.).

Die Therapie wird für mindestens 3 Monate durchgeführt; nach diesem Zeitraum wird die Fortsetzung der Therapie individuell entschieden, abhängig vom Erfolg und der Toleranz.

Bei mindestens 18, nach diesem Protokoll behandelten Patienten, hat sich das T4/T8-Verhältnis deutlich verbessert. Gleichzeitig konnten die klinischen Symptome nachhaltig über einen Zeitraum von wenigstens 6 Monaten gebessert werden. Als ein drastisches Beispiel der verblüffenden Wirkung einer Behandlung mit den erfindungsgemäßen Enzymmischungen, wie sie vorstehend geschildert wurde, sei der Krankheits- und Therapieverlauf des Patienten R. genannt. Der Patient R. wies ein T4/T8-Verhältnis von < 0,06 auf und galt bereits als hoffnungsloser Fall, der an die Dauerinfusion angeschlossen war. Nach der obengenannten Therapie (Therapiedauer Anfang Juli 1987 bis Mitte September 1987), verbesserte sich das T4/T8-Verhältnis auf > 0,1. Der Patient geht seit Ende August seiner normalen Arbeit nach und ist wieder voll sexuell aktiv.

### LITERATURLISTE

1. Goebel F.D., et al., Zur Pathophysiologie und Klinik des erworbenen Immundefektsyndroms (AIDS), Tempo Medical, Sonderheft Neueinführungen Dezember 1985 (1985), 8-13;
2. Jäger H., et al., Circulating Immune Complexes in HIV Infection, 2.International Symposium on Immunobiology in Clinical Oncology and Immune Dysfunctions, Nice, April 4-7, 1987;
3. Koch M., AIDS - vom Molekül zur Pandemie, Spektrum der Wissenschaft, Heidelberg, (1987);
4. Rieber E.P., Störungen der Immunantwort bei LAS und AIDS, Münch. Med. Wschr. 128 (1986) 463-466.

## Patentansprüche

1. Verwendung katabolischer Enzyme zur Herstellung eines Medikaments zum Bekämpfen der erworbenen Immunschwäche (AIDS) und deren Vorstadien (LAS, ARC).

2. Verwendung katabolischer Enzyme nach Anspruch 1,
**dadurch gekennzeichnet,**
daß die genannten Enzyme aus der Gruppe der proteolytischen oder hydrolytischen Enzyme ausgewählt werden.

3. Verwendung katabolischer Enzyme nach Anspruch 1
und/oder Anspruch 2,
**dadurch gekennzeichnet,**
daß mindestens ein Enzym verwendet wird, das ausgewählt wird aus einer Gruppe, enthaltend Papain, Pankreatin, Bromelin, Lipase, Amylase, Trypsin, α-Chymotrypsin und Serratia-Peptidase.

4. Verwendung katabolischer Enzyme nach mindestens einem der Ansprüche 1 bis 3,
**dadurch gekennzeichnet,**
daß eine Kombination von zwei oder mehreren der genannten Enzyme angewandt wird.

5. Verwendung katabolischer Enzyme nach mindestens einem der Ansprüche 1 bis 4,
**dadurch gekennzeichnet,**
daß zusätzlich Rutin und/oder Thymusfaktoren verwendet werden.

6. Verwendung katabolischer Enzyme nach mindestens einem der vorhergehenden Ansprüche,
**dadurch gekennzeichnet,**
daß zur Konfektionierung der Enzyme und gegebenenfalls weiteren Wirkstoffen übliche Hilfsstoffe, vorzugsweise Mannitol, zugegeben werden.

7. Verwendung katabolischer Enzyme nach Anspruch 6,
**dadurch gekennzeichnet,**
daß ein Gemisch von Enzymen verwendet wird, die aus fraktionierten Hydrolysaten von Rinderpankreas, Kalbsthymus, Pisum sativum und Lens esculenta gewonnen werden, sowie Papain und Mannitol, und zwar in den jeweiligen Mengen von 1-10, vorzugsweise 8 mg, 1-10, vorzugsweise 4 mg, 1-10, vorzugsweise 4 mg, 1-10, vorzugsweise 4 mg, 1-10, vorzugsweise 2 mg und 10-100, vorzugsweise 78 mg.

8. Verwendung katabolischer Enzyme nach mindestens einem der Ansprüche 1 bis 5,
**dadurch gekennzeichnet,**
daß ein Gemisch von 10-30, vorzugsweise 24 mg Trypsin, 1-10, vorzugsweise 1 mg α-Chymotrypsin, 40-100, vorzugsweise 60 mg Papain, 50-200, vorzugsweise 100 mg Pankreatin, 20-100, vorzugsweise 45 mg Bromelain, 5-50, vorzugsweise 10 mg Lipase, 5-50, vorzugsweise 10 mg Amylase sowie 10-100, vorzugsweise 50 mg Rutin angewendet wird.

9. Verwendung katabolischer Enzyme nach mindestens einem der Ansprüche 1 bis 5,
**dadurch gekennzeichnet,**
daß ein Gemisch von Trypsin, α-Chymotrypsin, Papain und Kalbsthymus in den jeweiligen Mengen von 1-10, vorzugsweise 4 mg, 1-10, vorzugsweise 4 mg, 5-50, vorzugsweise 10 mg und 1-10, vorzugsweise 3 mg in Ampullen oder jeweils 10-100, vorzugsweise 40 mg, 10-100, vorzugsweise 40 mg, 50-500, vorzugsweise 100 mg und 10-100, vorzugsweise 40 mg in Klistiertabletten oder oral einzunehmenden Tabletten verwendet werden.

## Claims

1. Use of catabolic enzymes for the preparation of a medicament for controlling acquired immune deficiency (AIDS) and its preliminary stages (LAS, ARC).

2. Use of catabolic enzymes according to Claim 1, characterised in that the said enzymes are selected from the group of proteolytic or hydrolytic enzymes.

3. Use of catabolic enzymes according to Claim 1 and/or Claim 2, characterised in that at least one enzyme which is selected from a group containing papain, pancreatin, bromelin [sic], lipase, amylase, trypsin, α-chymotrypsin and serratia peptidase is used.

4. Use of catabolic enzymes according to at least one of Claims 1 to 3, characterised in that a combination of two or more of the said enzymes is used.

5. Use of catabolic enzymes according to at least one of Claims 1 to 4, characterised in that rutin and/or thymus factors are additionally used.

6. Use of catabolic enzymes according to at least one of the preceding claims, characterised in that customary ancillary substances, preferably mannitol, are added to the final product of the enzymes and, where appropriate, other active substances.

7. Use of catabolic enzymes according to Claim 6, characterised in that a mixture of enzymes which are obtained from fractionated hydrolysates of bovine pancreas, calf thymus, Pisum sativum and Lens esculenta, as well as papain and mannitol, specifically in the respective amounts of 1-10, preferably 8 mg, 1-10, preferably 4 mg, 1-10, preferably 4 mg, 1-10, preferably 4 mg, 1-10, preferably 2 mg and 10-100, preferably 78 mg, is used.

8. Use of catabolic enzymes according to at least one of Claims 1 to 5, characterised in that a mixture of 10-30, preferably 24 mg of trypsin, 1-10, preferably 1 mg of α-chymotrypsin, 40-100, preferably 60 mg of papain, 50-200, preferably 100 mg of pancreatin, 20-100, preferably 45 mg of bromelain, 5-50, preferably 10 mg of lipase, 5-50, preferably 10 mg of amylase and 10-100, preferably 50 mg of rutin is used.

9. Use of catabolic enzymes according to at least one of Claims 1 to 5, characterised in that a mixture of trypsin, α-chymotrypsin, papain and calf thymus in the respective amounts of 1-10, preferably 4 mg, 1-10, preferably 4 mg, 5-50, preferably 10 mg and 1-10, preferably 3 mg in ampoules or in each case 10-100, preferably 40 mg, 10-100, preferably 40 mg, 50-500, preferably 100 mg and 10-100, preferably 40 mg in rectal tablets or tablets for oral intake are [sic] used.

## Revendications

1. Utilisation d'enzymes cataboliques pour la préparation d'un médicament pour la lutte contre des immunodéficiences acquises (SIDA) et leurs stades préliminaires (LAS,ARC).

2. Utilisation d'enzymes cataboliques selon la revendication 1, caractérisée en ce que lesdites enzymes sont choisies parmi les enzymes protéolytiques ou hydrolytiques.

3. Utilisation d'enzymes cataboliques selon la revendication 1 et/ou la revendication 2, caractérisée en ce que l'on utilise au moins une enzyme qui est choisie parmi la papaïne, la pancréatine, la broméline, la lipase, l'amylase, la trypsine, l'α-chymotrypsine et la peptidase de Serratia.

4. Utilisation d'enzymes cataboliques selon au moins l'une des revendications 1 à 3, caractérisée en ce que l'on utilise une association de deux des enzymes citées ou plus.

5. Utilisation d'enzymes cataboliques selon au moins l'une des revendications 1 à 4, caractérisée en ce que l'on utilise en plus la rutine et/ou des facteurs de thymus.

6. Utilisation d'enzymes cataboliques selon au moins l'une des revendications précédentes, caractérisée en ce que, pour la formulation des enzymes et éventuellement d'autres substances actives, on ajoute des substances auxiliaires usuelles, de préférence du mannitol.

7. Utilisation d'enzymes cataboliques selon la revendication 6, caractérisée en ce que l'on utilise un mélange d'enzymes qui sont obtenues à partir d'hydrolysats fractionnés de pancréas bovin, de thymus de veau, de Pisum sativum et Lens esculenta, ainsi que de la papaïne et du mannitol, à savoir, en les quantités respectives de 1-10, de préférence 8 mg, 1-10, de préférence 4 mg, 1-10, de préférence 4 mg, 1-10, de préférence 4 mg, 1-10, de préférence 2 mg, et 10-100, de préférence 78 mg.

8. Utilisation d'enzymes cataboliques selon au moins l'une des revendications 1 à 5, caractérisée en ce que l'on utilise un mélange de 10-30, de préférence 24 mg de trypsine, 1-10, de préférence 1 mg d'α-chymotrypsine, 40-100, de préférence 60 mg de papaïne, 50-200, de préférence 100 mg de pancréatine, 20-100, de préférence 45 mg de bromélaïne, 5-50, de préférence 10 mg de lipase, 5-50, de préférence 10 mg d'amylase, ainsi que 10-100, de préférence 50 mg de rutine.

9. Utilisation d'enzymes cataboliques selon au moins l'une des revendications 1 à 5, caractérisée en ce que l'on utilise un mélange de trypsine, d'α-chymotrypsine, de papaïne et de thymus de veau, en les quantités respectives de 1-10, de préférence 4 mg, 1-10, de préférence 4 mg, 5-50, de préférence 10 mg, et 1-10, de préférence 3 mg dans des ampoules, ou, respectivement, de 10-100, de préférence 40 mg, 10-100, de préférence 40 mg, 50-500, de préférence 100 mg et 10-100, de préférence 40 mg dans des comprimés pour lavement ou des comprimés à prendre par voie orale.
